# EUROPÄISCHE PATENTSCHRIFT

(11) **EP 1 265 656 B1**
(45) Veröffentlichungstag und Bekanntmachung des Hinweises auf die Patenterteilung: **30.04.2008**
(21) Anmeldenummer: 01915374.1
(22) Anmeldetag: 19.03.2001
(51) Int. Cl.: A61M 1/16, G06F 13/40, H04L 12/12, G06F 11/16

(54) **MEDIZINISCHES GERÄT MIT DOPPELTEM KOMMUNIKATIONSBUS**
MEDICAL APPLIANCE EQUIPPED WITH A DOUBLE COMMUNICATIONS BUS
APPAREIL MEDICAL A DOUBLE BUS DE COMMUNICATION

(30) Priorität: 20.03.2000 DE 10013665
(43) Veröffentlichungstag der Anmeldung: 18.12.2002
(73) Patentinhaber: Fresenius Medical Care Deutschland GmbH, 61352 Bad Homburg (DE)
(72) Erfinder: Manke, Joachim, 35792 Löhnberg (Niederhausen) (DE); Scheunert, Peter, 57290 Neunkirchen, Siegerl (DE)
(74) Vertreter: Laufhütte, Dieter
(86) Internationale Anmeldenummer: PCT/EP2001/003107
(87) Internationale Veröffentlichungsnummer: WO 2001/070301

(56) Entgegenhaltungen:
- WO-A-99/66407
- DE-C- 19 849 787
- US-A- 4 695 944
- US-A- 4 897 784

## Beschreibung

Die Erfindung betrifft ein medizinisches Gerät nach dem Oberbegriff des Anspruchs 1 sowie ein Verfahren zur Programmierung, der Durchführung von Testsequenzen, der Diagnose und der Wartung eines solchen Gerätes.

Derartige medizinische Geräte weisen oft eine Rechnerarchitektur mit zwei Kommunikationsbussen auf. Eine solche Rechnerarchitektur bietet sich im allgemeinen dann an, wenn ein medizinisches Gerät aufgrund von Sicherheitsbedürfnissen neben seinem Aktionsrechensystem ein Hilfsrechensystem benötigt, das die Funktionsweise des Aktionsrechensystems unabhängig überwacht und im Notfall einen sicheren Zustand des Gerätes herbeiführen kann.

Ein solches Gerät ist in dem deutschen Patent DE 198 49 787 C1 beschrieben, auf dessen Offenbarung hiermit ausdrücklich bezug genommen wird. In dieser Patentanmeldung wird ein Hämodialysegerät beschrieben, das - bezogen auf das Rechnersystem - einen modulartigen Aufbau aufweist. Eine Bedienungs-, eine Hydraulik- und eine Funktionseinheit beinhalten je einen Aktions- und einen Hilfsrechner. Die Aktionsrechner sind über einen Aktionsbus verbunden, die Hilfsrechner über einen Hilfsbus. Des weiteren ist eine Kommunikation zwischen dem Aktions- und dem Hilfsrechner innerhalb einer Einheit möglich.

Der Hilfsrechner der Bedieneinheit des Hämodialysegeräts weist eine externe Schnittstelle auf. Über diese Schnittstelle ist es möglich, mit Hilfe eines externen Gerätes die Programmierung, die Durchführung von Testsequenzen, die Diagnose und die Wartung aller Rechner des Hämodialysegerätes durchzuführen. Die Rechnerarchitektur dieses Gerätes erweist sich jedoch aus folgender Sicht als nachteilig. Während die Hilfsrechner relativ einfach über den Hilfsbus adressiert werden können, können die Aktionsrechner nur über die modulinterne Schnittstelle zwischen einem Aktions- und dem zugehörigen Hilfsrechner angesprochen werden. Erst nach einem solchen Übergang können die Daten über den Aktionsbus versendet werden. Die interne Rechnerverbindung erweist sich bzgl. der Datenübertragungsgeschwindigkeit als Engstelle, wodurch sich unnötig lange Zeiten bei der Datenkommunikation ergeben.

Gegen eine modulinterne Kommunikation bestehen aber auch Bedenken anderer Art. Sollen Daten auf einen Aktionsrechner übertragen werden, so ist es aus den besagten Gründen erforderlich, mindestens in einer Einheit die modulinterne Schnittstelle zwischen einem Aktions- und dem zugehörigen Hilfsrechner zu verwenden. Damit sind immer mehrere Rechner in den Datentransfervorgang eingebunden. Weiterhin ist in jedem Rechner zusätzliche Software notwendig, um den Datentransfer überhaupt handhaben zu können. Damit gestaltet sich der Vorgang insgesamt aufwendig und fehleranfällig.

Die DE-A-37 36 712 beschreibt ein ähnliches, modulartiges Dialysegerät, das eine hierarchische Rechnerstruktur vorsieht. Alle Steuerprozessoren sind an einen Hauptsteuerprozessor und alle Monitorprozessoren an einen Hauptmonitorprozessor verbunden. Die Hauptprozessoren sind wiederum untereinander verbunden.

Nach der EP 0 491 183 A1 ist ein Gerät bekannt, bei dem Treiber- und Empfängereinheiten zwischen einem Hauptdatenbus und einem internen Bus geschaltet sind. Damit zu Testzwecken zusätzlich vom Hauptdatenbus auf den internen Bus zugegriffen werden kann, sind bidrektionale Logikmittel vorgesehen, die jedoch einen komplizierten Aufbau aufweisen.

Die EP 0 306 211 A2 beschreibt ein sehr aufwendiges, synchronisiertes Zwillingsrechnerystem. Dabei sind zur Abstimmung der Taktabläufe sowie der parallel ablaufenden Programme eine Datenkommunikation zwischen zwei Rechnersystemen über eigene Controller vorgesehen.

In der DE 40 22 365 A1 wird ein Datenübertragungssystem vorgestellt, bei dem ein Adreß- und ein Datenbus zwischen einem Mikroprozessor und einem Datenspeicher zum Zwecke eines effizienteren Direktspeicherzugriffes (DMA) unterbrochen werden können.

Ein externes Gerät zur Diagnostik eines integrierten Schaltkreises über ein Bussystem ist Gegenstand der GB 2 282 244 A.

Der Erfindung liegt die Aufgabe zugrunde, ein gattungsgemäßes medizinisches Gerät derart weiterzubilden, daß eine einfache, schnelle und sichere Programmierung, Ablauf von Testsequenzen, Diagnose und/oder Wartung der an beiden Bussen befindlichen Endgeräte ermöglicht wird.

Nach der Lehre der Erfindung wird diese Aufgabe durch ein medizinisches Gerät nach dem Oberbegiff des Anspruches 1 dadurch gelöst, daß eine durch einen Schalter unterbrechbare Verbindungsleitung vorgesehen ist, durch die die beiden Kommunikationsbusse zu einem einheitlichen Kommunikationsbus verbindbar sind.

Mit Hilfe der erfindungsgemäßen Vorrichtung ist es möglich, die beiden Kommunikationsbusse immer dann, wenn ein externes Gerät zur Programmierung, zur Durchführung von Testsequenzen, zur Diagnose und/oder zur Wartung mit dem System verbunden werden soll, mit einer Verbindung der beiden Kommunikationsbusse zu einem einheitlichen Kommunikationsbus einfache, schnelle und sichere Verbindungswege zu schaffen, die nach diesen Verfahrensschritten wieder sicher und zuverlässig getrennt werden können.

Es ist auch denkbar, daß die Programmierung, die Durchführung von Testsequenzen, die Diagnose und/oder Wartung systemintern durch einen der im medizinischen Gerät vorhandenen Rechner durchgeführt wird. Hierbei wird dann ähnlich verfahren.

Der Schalter ist zweckmäßigerweise als Relaisschalter ausgebildet, es können aber auch andere Schalter, die eine einheitliche Verbindung zwischen den beiden Kommunikationsbussen ermöglichen, angewendet werden.

In einer besonders vorteilhaften Ausführungsform ist die Verbindungsleitung mit zwei in Serie angeordneten Schaltern unterbrechbar. Dabei kann der eine Schalter von einem Rechner, der mit dem ersten Kommunikationsbus in Verbindung steht, und der andere Schalter von einem Rechner, der mit dem zweiten Kommunikationsbus in Verbindung steht, betätigt werden.

In einer weiteren Ausführungsform sind Mittel zur Modusfeststellung vorgesehen, die eine Verbindung der Kommunikationsbusse immer dann ausschließen, wenn das medizinische Gerät seine eigentliche Funktion im Sinne einer medizinischen Anwendung, z.B. während einer Hämodialysebehandlung ausübt. Zweckmäßigerweise muß sich das Gerät bei dieser Ausführungsform zur Verbindung der beiden Kommunikationsbusse in einem bestimmten dafür vorgesehenen Modus befinden. Damit soll gewährleistet werden, daß das Sicherheitskonzept des medizinischen Gerätes, das u.a. auf den beiden getrennten Kommunikationsbussen aufbaut, nicht umgangen werden kann. Die Mittel zur Modusfeststellung sind dabei vorteilhafterweise Teil der Programmierung der beteiligten Rechner, die den oder die Schalter ansteuern.

Zur Verbindung mit einem externen Gerät kann das medizinische Gerät eine Schnittstelle direkt an einem der beiden Kommunikationsbusse, an einem der Rechner oder - im Falle einer Verbindungsleitung mit zwei Schaltern - in der Verbindungsleitung zwischen den beiden Schaltern aufweisen. In letzterem Falle ergibt sich ein besonders verläßlicher Ablauf bei der Verbindung mit einem externen Gerät, da jedes Rechnersystem, das über einen Kommunikationsbus verbunden ist, für sich steuern kann, ob es mit dem externen Gerät verbunden werden will.

Das externe Gerät kann durch einen weiteren Rechner oder ein Rechnernetzwerk realisiert werden, wobei die Verbindung aus einer lokalen Verbindung oder einer Intranet- oder Internetverbindung bestehen kann.

Es kann ein Detektormittel vorgesehen sein, das den Schalterzustand überprüft und an einen der Rechner weitermeldet.

Bei der erfindungsgemäßen Vorrichtung werden vorteilhafterweise Adressierungen verwendet, die ihre Eindeutigkeit im Falle der Busverbindung erhalten.

In einer besonders vorteilhaften Ausgestaltung der Erfindung finden CAN-Busse als Kommunikationsbusse Verwendung.

In einer weiteren Ausgestaltung der Erfindung weisen mindestens zwei Einheiten des medizinischen Gerätes je einen Aktions- und einen Hilfsrechner aus, wobei die Aktionsrechner über den ersten Kommunikationsbus und die Hilfsrechner über den zweiten Kommunikationsbus miteinander verbunden sind.

In einer ausgewählten Ausführungsform handelt es sich bei dem medizinischen Gerät um ein Blutbehandlungsgerät. An ein solches Gerät, das im allgemeinen einen extrakorporalen Blutkreislauf aufweist, bei dem Blut von einem Patienten oder einem Spender abgenommen, behandelt und wieder zurückgegeben wird, werden hohe Sicherheitsanforderungen gestellt, und es weist auch aus diesem Grunde einen komplexen Aufbau auf.

Als Blutbehandlungsverfahren seien u.a. die Blutreinigung durch Dialyse und/oder Filtration, durch Adsorption oder durch Zentrifugierung genannt.

Ein prinzipieller Aufbau eines für die Erfindung zu verwendenden medizinischen Gerätes wird in der DE 198 49 787 C1 beschrieben. Bei dem vorgestellten Dialysegerät ist eine Bedieneinheit zur Datenein- und ausgabe, eine Hydraulikeinheit zur Bereitstellung und Abführung der Dialysierflüssigkeit und des Ultrafiltrates sowie eine Funktionseinheit zur Steuerung des extrakorporalen Blutkreislaufs vorgesehen. Jede Einheit weist einen Aktions- und einen Hilfsrechner auf, wobei die Aktionsrechner über einen ersten Bus und die Hilfsrechner über einen zweiten Bus miteinander verbunden sind.

Ein solches Dialysegerät kann jedoch auch für die Hämofiltration vorgesehen werden, bei der die Hydraulikeinheit anstelle der Bereitstellung der Dialysierflüssigkeit die Bereitstellung von Substitutionsflüssigkeit übernimmt. Ähnliches gilt für ein Hämodiafiltrationsgerät, das Hämodialyse und Hämofiltration gleichermaßen ermöglicht, um die Vorteile der Behandlungseffizienz beider Verfahren zu kombinieren.

Der Erfindung liegt auch die Aufgabe zugrunde, ein Verfahren für einen einfachen, schnellen und sicheren Programmier-, Test-, Diagnose- und/oder Wartungsvorgang eines gattungsgemäßen medizinisches Gerätes mit einer Schnittstelle zu einem externen Gerät bereitzustellen.

Die Aufgabe wird dadurch gelöst, daß zu diesem Zweck ein externes Gerät an die dafür vorgesehene Schnittstelle angeschlossen wird, die beiden Kommunikationbusse zu einem einheitlichen Kommunikationsbus verbunden werden, der Programmier-, Test-, Diagnostizier- und/oder Wartungsvorgang durch das externe Gerät durchgeführt wird, und nach Abschluß des Programmier-, Test-, Diagnostizier- und/oder Wartungsvorgangs die Kommunikationsbusse wieder getrennt werden.

Weitere Einzelheiten und Vorteile der Erfindung werden anhand eines in der Zeichnung dargestellten Ausführungsbeispiels näher beschrieben. Es zeigt:

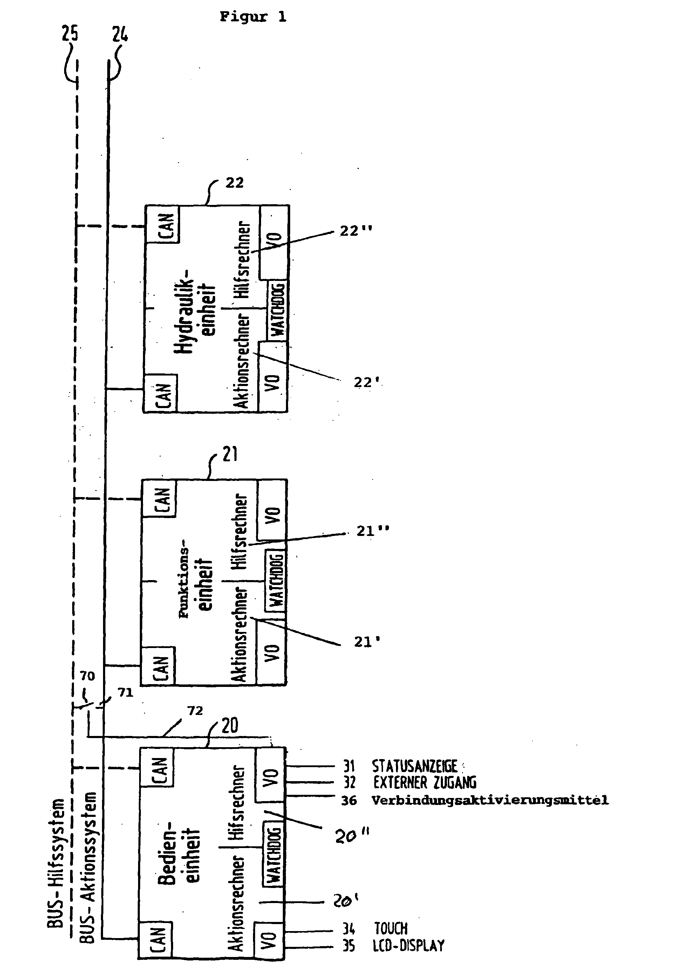

Figur 1: eine Rechnerstruktur eines erfindungsgemäßen Hämodialysegerätes.

Die Figur 1 zeigt die Rechnerstruktur eines Hamodialysegerätes gemäß der DE 198 49 787 C1. Soweit dies zum Verständnis der Erfindung nicht unbedingt erforderlich ist, wurde der Übersichtlichkeit halber auf die Darstellung der Aktoren und Sensoren der einzelnen Einheiten verzichtet. Derartige Details sind aber in der DE 198 49 787 C1 offenbart.

Die Rechnerstruktur der Hämodialysemaschine weist eine Bedieneinheit 20 mit einem Aktionsrechner 20' mit einem Touchscreen 34 als Eingabemittel und einem LCD-Display 35 als Ausgabemittel auf. Des weiteren beinhaltet die Bedieneinheit einen Hilfsrechner 20", mit dem eine Statusanzeige 31, ein externer Zugang 32 und ein Verbindungsaktivierungsmittel 36 verbunden sind.

Eine Funktionseinheit 21 ist ebenfalls aus einem Aktionsrechner 21' und einem Hilfsrechner 21" zusammengesetzt. Diese Rechner steuern und überwachen den extrakoporalen Blutkreislauf des Hämodialysegerätes. Auch die Hydraulikeinheit 22, die die Aufbereitung, den Zu- und Abfluß der Dialysierflüssigkeit sowie die Ultrafiltration regelt und überwacht, ist in einen Aktionsrechner 22' und einen Hilfsrechner 22" aufgeteilt.

Die Aktionsrechner 20', 21' und 22' sind über einen Bus "Aktionssystem" 24 und die Hilfsrechner 20'', 21'' und 22" über einen Bus "Hilfssystem" 25 miteinander verbunden. Des weiteren ist eine Verbindungsleitung 71 zur Verbindung der Busse 24 und 25 vorgesehen, die mit einem Relaisschalter 70 verbunden bzw. unterbrochen werden kann.

Der Relaisschalter 70 ist über eine Leitung 72 mit dem Hilfsrechner 20" der Bedieneinheit 20 verbunden. Soll nun ein Programmier-, Test-, Diagnostizier- und/oder Wartungsvorgang für das gesamte System oder Teile des Systems beginnen, so betätigt der Benutzer die Verbindungsaktivierungsmittel 36. Die Programmierung des Hilfsrechners 20" umfaßt dabei nicht näher gezeigte Mittel zur Modusfeststellung, die den Status der Hämodialysemaschine erfassen und die Verbindungsaktivierungsmittel 36 nur freigeben, wenn die Hämodialysemaschine in einem entsprechenden Service-Modus ist, nicht aber während einer Dialysebehandlung. In letzterem Fall soll die Trennung der Busse 24 und 25 aus Sicherheitsgründen beibehalten werden.

Nach der Betätigung der Verbindungsaktivierungsmittel 36 gibt der Hilfsrechner 20' ein Signal auf die Leitung 72, um den Relaisschalter 70 zu schließen. Der nun eingeleitete Vorgang kann über die Statusanzeige 31 an den Benutzer bestätigt werden.

Ein externes Gerät, das an den externen Zugang 32 angeschlossen wird, hat nun über die CAN-Schnittstelle 73 des Hilfsrechners 20' direkten Zugriff auf alle Rechner der Einheiten 20, 21 und 22, da die Busse 24 und 25 miteinander verbunden sind.

Ist der Programmier-, Test-, Diagnostizier- und/oder Wartungsvorgang beendet, so wird über die Verbindungsaktivierungsmittel 36 der Schalter 70 wieder geöffnet und die Verbindungsleitung 71 unterbrochen. Auf diese Weise können das Aktions- und das Hilfssystem ihre eigentlich unabhängige Funktion getrennt fortführen.

Die Verbindung 71 ist in Figur 1 symbolisch an einer willkürlichen Stelle der Busse 24 und 25 gezeichnet. Je nach verwendetem Bussystem sind dem Fachmann die Umstände geläufig, wie Trennungen und Verbindungen in bestehende Busleitungen - ggf. unter Beachtung von Abschlußwiderständen - durchgeführt werden können, ohne die Busfunktionen zu beeinflußen.

## Patentansprüche

1. Medizinisches Gerät
mit mindestens zwei Einheiten (20, 21, 22) zur Überwachung und/oder Steuerung des medizinischen Gerätes, die hierzu je mindestens einen Rechner (20', 21', 22') mit einem Mikroprozessor aufweisen,
wobei mindestens eine Einheit einen zweiten Rechner (20", 21 ", 22") mit einem zweiten Mikroprozessor beinhaltet,
mit zwei Kommunikationsbussen (24, 25),
wobei jeder der Rechner entweder an den ersten oder an den zweiten Kommunikationsbus angeschlossen ist,
**dadurch gekennzeichnet, daß**
eine durch einen Schalter (70) unterbrechbare Verbindungsleitung (71) vorgesehen ist,
durch die die beiden Kommunikationsbusse zu einem einheitlichen Kommunikationsbus verbindbar sind.

2. Medizinisches Gerät nach Anspruch 1, **dadurch gekennzeichnet, daß** der Schalter als Relaisschalter ausgebildet ist.

3. Medizinisches Gerät nach einem der Ansprüche 1 oder 2, **dadurch gekennzeichnet, daß** der Schalter durch einen der Rechner angesteuert werden kann.

4. Medizinisches Gerät nach einem der Ansprüche 1 bis 3, **dadurch gekennzeichnet, daß** die Verbindungsleitung durch zwei in Serie angeordneten Schaltern unterbrechbar ist.

5. Medizinisches Gerät nach Anspruch 4, **dadurch gekennzeichnet, daß** einer der Schalter von einem Rechner, der mit dem ersten Kommunikationsbus in Verbindung steht, und der andere Schalter von einem Rechner, der mit dem zweiten Kommunikationbus in Verbindung steht, betätigt werden.

6. Medizinisches Gerät nach einem der Ansprüche 1 bis 5, **dadurch gekennzeichnet, daß** Mittel zur Modusfeststellung vorgesehen sind, die ein Verbinden des ersten und des zweiten Kommunikationsbusses mit Hilfe des Schalters nur dann erlauben, wenn das Gerät in einem speziellen dafür vorgesehen Modus ist.

7. Medizinisches Gerät nach einem der Ansprüche 1 bis 6, **dadurch gekennzeichnet, daß** die Kommunikationsbusse CAN-Busse sind.

8. Medizinisches Gerät nach einem der Ansprüche 1 bis 7, **dadurch gekennzeichnet, daß** in beiden Kommunikationsbussen Adressierungen verwendet werden, die eine eindeutige Adressierung der Rechner und ihrer Komponenten bei Verbindung der Kommunikationsbusse erlauben.

9. Medizinisches Gerät nach einem der Ansprüche 1 bis 8, **dadurch gekennzeichnet, daß** einer der Kommunikationsbusse eine Schnittstelle zur Verbindung mit einem externen Gerät aufweist.

10. Medizinisches Gerät nach einem der Ansprüche 1 bis 8, **dadurch gekennzeichnet, daß** einer der Rechner eine Schnittstelle (32) zur Verbindung mit einem externen Gerät aufweist.

11. Medizinisches Gerät nach Anspruch 4, **dadurch gekennzeichnet, daß** eine Schnittstelle mit einem externen Gerät in der Verbindungsleitung zwischen den beiden Schaltern vorgesehen ist.

12. Medizinisches Gerät nach einem der Ansprüche 9 bis 11, **dadurch gekennzeichnet, daß** einzelne oder alle Rechner des medizinischen Gerätes mit Hilfe des externen Gerätes programmiert, getestet, diagnostiziert und/oder gewartet werden können.

13. Medizinisches Gerät nach Anspruch 1 bis 12, **dadurch gekennzeichnet, daß** die mindestens zwei Einheiten (20, 21, 22) je einen Aktionsrechner (20', 21', 22') und einen Hilfsrechner (20", 21 ", 22") aufweisen.

14. Medizinisches Gerät nach Anspruch 13, **dadurch gekennzeichnet, daß** die Aktionsrechner über den ersten Kommunikationsbus (24) und die Hilfsrechner über den zweiten Kommunikationsbus (25) miteinander verbunden sind.

15. Medizinisches Gerät nach Anspruch 1 bis 14, **dadurch gekennzeichnet, daß** das medizinische Gerät ein Blutbehandlungsgerät ist.

16. Medizinisches Gerät nach Anspruch 15, **dadurch gekennzeichnet, daß** das Blutbehandlungsgerät ein Hämodialyse-, Hämofiltrations- oder Hämdiafiltrationsgerät ist.

17. Medizinisches Gerät nach Anspruch 16, **dadurch gekennzeichnet, daß** die Einheiten eine Bedieneinheit (20) zur Datenein- und -ausgabe, eine Hydraulikeinheit (21) zur Bereitstellung und/oder Abführung von biologischen Flüssigkeiten und eine Funktionseinheit (22) zur Steuerung eines extrakorporalen Blutkreislaufes umfassen.

18. Verfahren zur Durchführung eines Programmier-, Test-, Diagnose- und/oder Wartungsvorganges eines medizinisches Gerätes mit mindestens zwei Einheiten zur Überwachung und/oder Steuerung des medizinischen Gerätes, die hierzu je mindestens einen Rechner mit einem Mikroprozessor aufweisen, wobei mindestens eine Einheit einen zweiten Rechner mit einem zweiten Mikroprozessor beinhaltet, mit zwei Kommunikationsbussen, wobei jeder der Rechner entweder an den ersten oder an den zweiten Kommunikationsbus angeschlossen ist, mit einer Schnittstelle zu einem externen Gerät, die an einem der Rechner oder einem der Kommunikationsbusse angeschlossen ist,
**dadurch gekennzeichnet, daß**
zu diesem Zweck ein externes Gerät an die dafür vorgesehene Schnittstelle angeschlossen wird,
die beiden Kommunikationsbusse zu einem einheitlichen Kommunikationsbus verbunden werden,
der Programmier-, Test-, Diagnostizier- und/oder Wartungsvorgang durch das externe Gerät durchgeführt wird, und
nach Abschluß des Programmier-, Test-, Diagnostizier- und/oder Wartungsvorgangs die Kommunikationsbusse wieder getrennt werden.

19. Verfahren nach Anspruch 18, **dadurch gekennzeichnet, daß** vor dem Verbinden der beiden Kommunikationsbusse der Modus des medizinischen Gerätes festgestellt wird und das Verbinden davon abhängig gemacht wird, ob sich das Gerät in einem speziellen Status zur Durchführung des Programmier-, Test-, Diagnostizier- und/oder Wartungsvorgangs befindet.

## Claims

1. A medical device comprising
at least two units (20, 21, 22) for monitoring and/or controlling the medical device, which for that purpose have at least one respective computer (20', 21', 22') with a microprocessor,
wherein at least one unit has a second computer (20", 21", 22") with a second microprocessor,
two communication buses (24, 25),
wherein each of the computers is connected either to the first or the second communication bus,
**characterised in that**
there is provided a connecting line (71) which can be interrupted by a switch (70) and
by means of which the two communication buses can be connected to form a unitary communication bus.

2. A medical device according to claim 1 **characterised in that** the switch is in the form of a relay switch.

3. A medical device according to one of claims 1 and 2 **characterised in that** the switch can be actuated by means of one of the computers.

4. A medical device according to one of claims 1 to 3 **characterised in that** the connecting line can be interrupted by two switches arranged in series.

5. A medical device according to claim 4 **characterised in that** one of the switches is actuated by a computer connected to the first communication bus and the other switch is actuated by a computer connected to the second communication bus.

6. A medical device according to one of claims 1 to 5 **characterised in that** there are provided means for mode establishment which allow the first and the second communication buses to be connected together by means of the switch only when the device is in a special mode provided for that purpose.

7. A medical device according to one of claims 1 to 6 **characterised in that** the communication buses are CAN buses.

8. A medical device according to one of claims 1 to 7 **characterised in that** both communication buses use addressing processes which allow unique addressing of the computers and their components when the communication buses are connected.

9. A medical device according to one of claims 1 to 8 **characterised in that** one of the communication buses has an interface for connection to an external device.

10. A medical device according to one of claims 1 to 8 **characterised in that** one of the computers has an interface (32) for connection to an external device.

11. A medical device according to claim 4 **characterised in that** there is provided an interface to an external device in the connecting line between the two switches.

12. A medical device according to one of claims 9 to 11 **characterised in that** individual or all computers of the medical device can be programmed, tested, diagnosed and/or maintained by means of the external device.

13. A medical device according to claims 1 to 12 **characterised in that** the at least two units (20, 21, 22) each have an action computer (20', 21', 22') and an auxiliary computer (20", 21 ", 22").

14. A medical device according to claim 13 **characterised in that** the action computers are connected together by means of the first communication bus (24) and the auxiliary computers are connected together by means of the second communication bus (25).

15. A medical device according to claims 1 to 14 **characterised in that** the medical device is a blood treatment device.

16. A medical device according to claim 15 **characterised in that** the blood treatment device is a haemodialysis, haemofiltration or haemodiafiltration device.

17. A medical device according to claim 16 **characterised in that** the units include an operating unit (20) for data input and output, a hydraulic unit (21) for providing and/or draining off biological fluids and a functional unit (22) for controlling an extracorporeal blood circulation.

18. A method of carrying out a programming, test, diagnosis and/or maintenance operation of a medical device comprising at least two units for monitoring and/or controlling the medical device which for that purpose have at least one respective computer with a microprocessor, wherein at least one unit contains a second computer with a second microprocessor, comprising two communication buses, where each of the computers is connected either to the first or the second communication bus, and comprising an interface to an external device, which is connected to one of the computers or one of the communication buses,
**characterised in that**
for that purpose an external device is connected to the interface provided for that purpose,
the two communication buses are connected to form a unitary communication bus,
the programming, test, diagnostic and/or maintenance operation is carried out by the external device, and
after conclusion of the programming, test, diagnostic and/or maintenance operation the communication buses are separated again.

19. A method according to claim 18 **characterised in that** prior to the connection of the two communication buses the mode of the medical device is established and the connection is made in dependence on whether the device is in a special state for carrying out the programming, test, diagnostic and/or maintenance operation.

## Revendications

1. Appareil médical
avec au moins deux unités (20, 21, 22) pour la surveillance et/ou la commande de l'appareil médical, qui présentent à cette fin chacune au moins un calculateur (20', 21', 22') avec un microprocesseur,
où au moins une unité contient un deuxième calculateur (20", 2"', 22") avec un deuxième microprocesseur,
avec deux bus de communication (24, 25),
où chacun des calculateurs est connecté soit au premier soit au deuxième bus de communication,
**caractérisé en ce qu'**une ligne de liaison (71) pouvant être interrompue par un commutateur (70) est prévue
par laquelle les deux bus de communication peuvent être reliés en un bus de communication unitaire.

2. Appareil médical selon la revendication 1, **caractérisé en ce que** le commutateur est réalisé comme commutateur relais.

3. Appareil médical selon l'une des revendications 1 ou 2, **caractérisé en ce que** le commutateur peut être commandé par l'un des calculateurs.

4. Appareil médical selon l'une des revendications 1 à 3, **caractérisé en ce que** la ligne de liaison peut être interrompue par deux commutateurs montés en série.

5. Appareil médical selon la revendication 4, **caractérisé en ce que** l'un des commutateurs est actionné par un calculateur, qui est en liaison avec le premier bus de communication, et l'autre commutateur par un calculateur qui est en liaison avec le deuxième bus de communication.

6. Appareil médical selon l'une des revendications 1 à 5, **caractérisé en ce que** des moyens pour la constatation du mode sont prévus, qui permettent une liaison du premier et du deuxième bus de communication à l'aide du commutateur seulement lorsque l'appareil se trouve dans un mode spécial prévu à cette fin.

7. Appareil médical selon l'une des revendications 1 à 6, **caractérisé en ce que** les bus de communication sont des bus CAN.

8. Appareil médical selon l'une des revendications 1 à 7, **caractérisé en ce que** dans les deux bus de communication, des adressages sont utilisés qui permettent un adressage sans ambiguité des calculateurs et de leurs composants lors de la liaison des bus de communication.

9. Appareil médical selon l'une des revendications 1 à 8, **caractérisé en ce que** l'un des bus de communication présente une interface pour la connexion avec un appareil externe.

10. Appareil médical selon l'une des revendications 1 à 8, **caractérisé en ce que** l'un des calculateurs présente une interface (32) pour la connexion avec un appareil externe.

11. Appareil médical selon la revendication 4, **caractérisé en ce qu'**une interface avec un appareil externe est prévue dans la ligne de liaison entre les deux commutateurs.

12. Appareil selon l'une des revendications 9 à 11, **caractérisé en ce que** des calculateurs individuels ou bien tous les calculateurs de l'appareil médical peuvent être programmés, testés, diagnostiqués et/ou entretenus à l'aide de l'appareil externe.

13. Appareil médical selon la revendication 1 à 12, **caractérisé en ce qu'**au moins deux unités précitées (20, 21, 22) présentent chacune un calculateur régissant les actions (20', 21', 22') et un calculateur auxiliaire (20", 21", 22").

14. Appareil médical selon la revendication 13, **caractérisé en ce que** les calculateurs régissant les actions sont reliés entre eux par le premier bus de communication (24) et les calculateurs auxiliaires par le deuxième bus de communication (25).

15. Appareil médical selon la revendication 1 à 14, **caractérisé en ce que** l'appareil médical est un appareil de traitement du sang.

16. Appareil médical selon la revendication 15, **caractérisé en ce que** l'appareil de traitement de sang est un appareil d'hémodialyse, d'hémofiltration ou d'hémodiafiltration.

17. Appareil médical selon la revendication 16, **caractérisé en ce que** les unités comprennent une unité de commande (20) pour l'entrée et l'émission des données, une unité hydraulique (21) pour la mise à disposition et/ou l'évacuation de liquides biologiques et une unité fonctionnelle (22) pour commander une circulation sanguine extracorporelle.

18. Procédé pour exécuter une opération de programmation, de test, de diagnostic et/ou d'entretien d'un appareil médical avec au moins deux unités pour la surveillance et/ou la commande d'un appareil médical, qui présentent à cette fin chacune au moins un calculateur avec un microprocesseur, où au moins une unité contient un deuxième calculateur avec un deuxième microprocesseur, avec deux bus de communication, où chacun des calculateurs est connecté soit au premier soit au deuxième bus de communication, avec une interface à un appareil externe, qui est reliée à l'un des calculateurs ou l'un des bus de communication,
**caractérisé en ce que**
à cette fin un appareil externe est connecté à l'interface prévue à cette fin,
les deux bus de communication sont reliés en un bus de communication unitaire,
l'opération de programmation, de test, de diagnostic et/ou d'entretien est exécutée par l'appareil externe, et
après l'achèvement de l'opération de programmation, de test, de diagnostic et/ou d'entretien, les bus de communication sont séparés à nouveau.

19. Procédé selon la revendication 18, **caractérisé en ce qu'**avant la connexion des deux bus de communication, le mode de l'appareil médical est constaté, et la connexion dépendra du fait si l'appareil se trouve dans un état spécial pour l'exécution de l'opération de programmation, de test, de diagnostic et/ou d'entretien.
